# EUROPÄISCHE PATENTSCHRIFT

(11) **EP 2 004 097 B1**
(45) Veröffentlichungstag und Bekanntmachung des Hinweises auf die Patenterteilung: **23.04.2014**
(21) Anmeldenummer: 07723064.7
(22) Anmeldetag: 06.03.2007
(51) Int. Cl.: A61F 2/34, A61F 2/40

(54) **GELENKPFANNE, INSBESONDERE FÜR EINE HÜFTENDOPROTHESE**
JOINT SOCKET, IN PARTICULAR FOR A HIP ENDOPROSTHESIS
CAVITE GLENOÏDE, EN PARTICULIER POUR UNE ENDOPROTHESE DE HANCHE

(30) Priorität: 13.04.2006 DE 102006017473
(43) Veröffentlichungstag der Anmeldung: 24.12.2008
(73) Patentinhaber: Smith & Nephew Orthopaedics AG, 6343 Rotkreuz (CH)
(72) Erfinder: IMHOF, Martin, CH-6045 Meggen (CH)
(74) Vertreter: Popp, Eugen
(86) Internationale Anmeldenummer: PCT/EP2007/001919
(87) Internationale Veröffentlichungsnummer: WO 2007/118553

(56) Entgegenhaltungen:
- EP-A1- 0 169 978
- EP-A1- 0 663 193
- EP-A2- 0 144 588
- DE-A1- 4 337 936
- DE-A1- 10 360 390

## Beschreibung

Die Erfindung betrifft eine Gelenkpfanne, insbesondere für eine Hüftendoprothese, mit einer Pfannenschale, und einem Pfanneneinsatz zur Lagerung eines Gelenkkopfes, gemäß dem Oberbegriff des Anspruches 1, wie sie aus der DE 103 60 390 A1 als bekannt hervorgeht.

Bei Totalendoprothesen, insbesondere Hüfttotalendoprothesen, wird in den Femur ein Prothesenschaft mit einem Gelenkkopf eingesetzt. In den Beckenknochen wird eine Gelenkpfanne implantiert, die als Lagerschale für den Gelenkkopf dient. Die Gelenkpfanne umfaßt in der Regel eine Pfannenschale und einen Pfanneneinsatz. Die Pfannenschale wird im Bezug auf den Knochen optimiert, während der Pfanneneinsatz für die Lagereigenschaften des Gelenkkopfes maßgebend ist. Die Pfannenschale ist so gestaltet und im Knochen positioniert, daß ein möglichst stabiles Einwachsen der Pfannenschale in den Knochen möglich ist. Der Pfanneneinsatz kann in der Pfannenschale so ausgerichtet werden, daß der Gelenkkopf mit möglichst korrekter orthopädischer Lage des Prothesenschaftes und damit Femurs des Patienten aufgenommen wird.

Aus der EP 0 663 193 A1 ist eine Gelenkpfanne bekannt, bei welcher der Pfanneneinsatz eine sphärische Außenfläche aufweist und mit dieser sphärischen Außenfläche in einem sphärischen Aufnahmeraum mit gleichem Kugelradius der Pfannenschale sitzt. Beim Einsetzen des Pfanneneinsatzes in die Pfannenschale kann der Pfanneneinsatz beliebig um seine Rotationsachse gedreht und mit seiner Rotationsachse beliebig gegenüber der Rotationsachse des Aufnahmeraumes gekippt werden. Dadurch ist es möglich, die Pfannenschale im Knochen entsprechend der Knochenstruktur zu positionieren. Der Pfanneneinsatz kann entsprechend der orthopädischen Lage des Prothesenschaftes und Gelenkkopfes ausgerichtet werden. Um den Pfanneneinsatz in seiner Lage in der Pfannenschale zu fixieren, weist die sphärische Innenfläche des Aufnahmeraumes der Pfannenschale spitz vorstehende Zähne auf, die in die Außenfläche des Pfanneneinsatzes eingreifen. Da die Zähne in die Außenfläche des Pfanneneinsatzes eindringen müssen, bestehen Beschränkungen in bezug auf die Wahl des Materials des Pfanneneinsatzes. Das Einpressen des Pfanneneinsatzes auf die Zähne der Pfannenschale erschwert das exakt positionierte Einsetzen des Pfanneneinsatzes.

Um die vorgenannte Problematik zu vermeiden, ist in der DE 103 60 390 A1 vorgeschlagen, daß die Außenfläche des Pfanneneinsatzes die Innenfläche des Aufnahmeraumes in einer zur Rotationsachse des Aufnahmeraumes konzentrischen Berührungslinie berührt, daß die Innenfläche des Aufnahmeraumes sich in dem Bereich dieser Berührungslinie gegen den Pol des Aufnahmeraumes in der Weise verengt, daß der Krümmungsradius in diesem Bereich stets größer ist als der Kugelradius der Außenfläche des Pfanneneinsatzes, und daß der Pfanneneinsatz in dem Aufnahmeraum selbsthemmend klemmbar ist. Aufgrund der vorgenannten linienförmigen Berührung zwischen Pfanneneinsatz und Pfannenschale ist ein leichtes Drehen und Kippen des Pfanneneinsatzes in dem Aufnahmeraum der Pfanneschale möglich, um den Pfanneneinsatz optimal in seiner Lage auszurichten. Sobald der Pfanneneinsatz ausgerichtet ist, genügt ein leichter Druck, um den Pfanneneinsatz in den sich verengenden Aufnahmeraum einzupressen, worauf der Pfanneneinsatz in dem Aufnahmeraum selbsthemmend geklemmt wird. Die selbsthemmende Klemmung bewirkt eine Fixierung des Pfanneneinsatzes in der Pfannenschale mit hoher Stabilität. Eine Belastung des Gelenkes bewirkt dabei ein zusätzliches Einpressen des Pfanneneinsatzes in die Pfannenschale, so daß die Fixierung der Pfannenschale zusätzlich verstärkt wird. Da sich die Fixierung des optimal ausgerichteten Pfanneneinsatzes durch einfaches Eindrücken in dem Aufnahmeraum ergibt, ist diese Fixierung einfach durchführbar und erfordert keine zusätzlichen Instrumente oder zusätzliche Befestigungsmittel. Die selbsthemmende Klemmung stellt sich bei einem minimalen Verschiebungsweg des Pfanneneinsatzes in den Aufnahmeraum ein, so daß bei dem Fixieren des Pfanneneinsatzes keine unbeabsichtigte Dejustage der Ausrichtung des Pfanneneinsatzes auftreten kann. Bezüglich weiterer Vorteile der vorgenannten Konstruktion wird auf die DE 103 60 390 A1 verwiesen. Dies gilt insbesondere auch für den Vorteil, daß bei einem Anschlagen des Schenkelhalses eine Prothesenschaftes am Rand der Gelenkpfanne im ungünstigsten Fall nur der Pfanneneinsatz in der Pfannenschale gelockert wird, jedoch nicht die Pfannenschale im Knochen. Bei einer anschließenden regulären Belastung des Gelenkes wird der Pfanneneinsatz dann wieder in den Aufnahmeraum der Pfannenschale eingepreßt und erneut festgeklemmt und fixiert.

Der vorliegenden Erfindung liegt nun die Aufgabe zugrunde, ausgehend vom zuletzt genannten Stand der Technik und unter Beibehaltung der Vorteile desselben eine Gelenkpfanne, insbesondere eine Gelenkpfanne für eine Hüftendoprothese, zur Verfügung zu stellen, die eine erhöhte Klemmwirkung zwischen Pfannenschale und Pfanneneinsatz gewährleistet, wobei gleichzeitig darauf geachtet ist, daß der spezifische Druck zwischen den miteinander korrespondierenden Klemmflächen reduziert ist.

Diese Aufgabe wird erfindungsgemäß durch die kennzeichnenden Merkmale des Anspruches 1 gelöst. Dadurch ist es möglich, die Kontaktfläche der miteinander korrespondierenden Klemmflächen zu erhöhen und den spezifischen Druck im Bereich der miteinander korrespondierenden Klemmflächen zu reduzieren, ohne daß die Klemmwirkung darunter leidet.

Ein wesentlicher Punkt der vorliegenden Erfindung ist also, daß der Verformungswiderstand wenigstens einer der miteinander korrespondierenden Flächen von Aufnahmeraum und Pfanneneinsatz gegenüber demjenigen des Kernbereichs dieser Teile durch Ausbildung einer ausgeprägten Oberflächenstruktur erniedrigt ist. Dadurch wird eine erhöhte Verformbarkeit wenigstens einer der miteinander korrespondierenden Klemmflächen erhalten, wodurch die Kontaktfläche erhöht und der spezifische Druck entsprechend reduziert wird, ohne daß dadurch die Klemmwirkung nachteilig beeinflußt ist.

Die erfindungsgemäße Oberflächenstruktur wird bei einer bevorzugten Ausführungsform dadurch erhalten, daß die entsprechende Oberfläche sich über den Umfang von Aufnahmeraum und/oder Pfanneneinsatz erstreckende Rippen oder dgl. Erhebungen aufweist. Vorzugsweise sind die Rippen im Querschnitt dreieckförmig bzw. spitzdachartig ausgebildet.

Entsprechend dem Stand der Technik gemäß der DE 103 60 390 A1 kann die Innenfläche des Aufnahmeraumes im Bereich der Berührungslinie konisch ausgebildet sein, d.h. mit einem unendlichen Krümmungsradius.

Der Kegelwinkel der Innenfläche liegt dabei zwischen etwa 3,5° und 12°, insbesondere zwischen etwa 5° und 8°. Dabei handelt es sich um einen bevorzugten Bereich für einen sogenannten "Selbsthemmungswinkel", der von der Materialpaarung abhängt. Es handelt sich um den Winkel zwischen der Konusmittelachse und der Konusmantellinie.

Aufgrund der Oberflächenstruktur wenigstens einer der miteinander korrespondierenden Flächen ist es möglich, Pfanneneinsatz und Pfannenschale jeweils aus einem harten Werkstoff zu fertigen. Durch die Oberflächenstruktur wird auch dann der erfindungsgemäß geforderte geringere Verformungswiderstand dieser Fläche erhalten. Die Pfannenschale wird vorzugsweise aus einem biokompatiblen Metall hergestellt, z.B. einer Titan-Legierung. Für den Pfanneneinsatz kann ein Werkstoff entsprechend der Gleitpaarung von Pfannenschale und Gelenkkopf gewählt werden, z.B. ein metallischer oder keramischer Werkstoff oder auch Kunststoff.

Abhängig von den Metallpaarungen kann es vorteilhaft sein, daß die Umfangsrippen oder -erhebungen jeweils über den Umfang gleichförmig verteilt unterbrochen sind.

Eine besonders vorteilhafte Ausführungsform zeichnet sich dadurch aus, daß die Oberflächenstruktur der wenigstens einen der miteinander korrespondierenden Flächen aus im Querschnitt dreieckigen Rillen besteht, die von in Umfangsrichtung verlaufenden Rippen mit ebenfalls dreieckigem oder gegebenenfalls auch trapezförmigem Querschnitt getrennt sind.

Nachstehend wird eine bevorzugte Ausführungsform einer erfindungsgemäß ausgebildeten Gelenkpfanne bzw. ein Teil derselben anhand der beigefügten Zeichnung näher beschrieben. Es zeigen:
- Fig. 1: eine Hüfttotalendoprothese, teilweise im Axialschnitt, teilweise in Seitenansicht;
- Fig. 2: einen Teil der Gelenkpfanne der Prothese gemäß Fig. 1 im Axialschnitt;
- Fig. 3: einen vergrößerten Ausschnitt III der Gelenkpfanne gemäß Fig. 2 in vergrößertem Maßstab; und
- Fig. 4: den Ausschnitt gemäß Fig. 3 in noch weiter vergrößertem Maßstab.

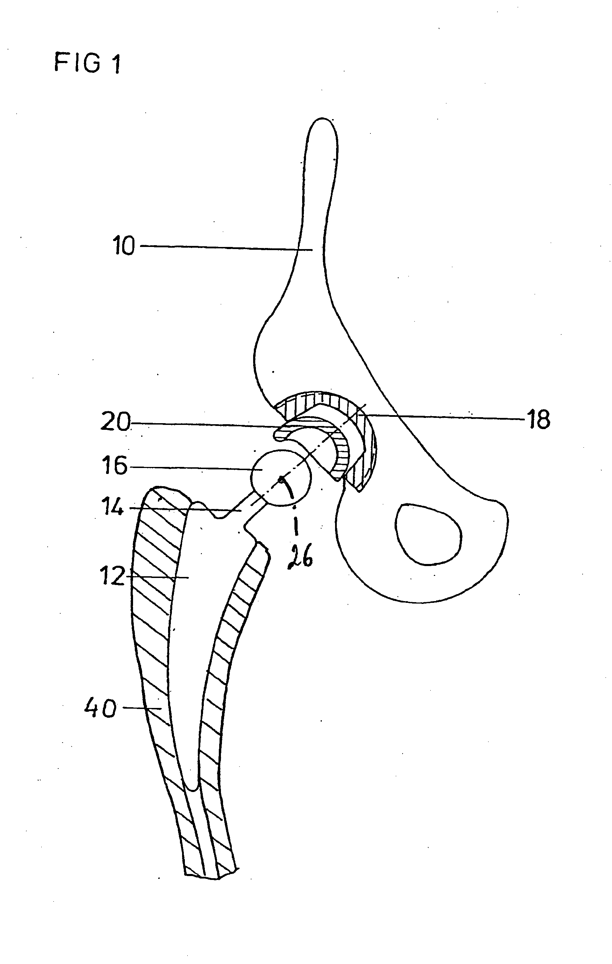

Die in Fig. 1 schematisch dargestellte Hüfttotalendoprothese besteht aus einer in einen Beckenknochen 10 implantierbaren Gelenkpfanne und einem Prothesenschaft 12, der in den Femur 40 eingesetzt ist. Der Prothesenschaft 12 weist einen Schenkelhals 14 auf, auf welchem ein Gelenkkopf 16 angeordnet ist, der in der Gelenkpfanne gelagert wird. Die Gelenkpfanne besteht aus einer im Beckenknochen 10 positionierbaren Pfannenschale 18 und einem Pfanneneinsatz 20. Die Pfannenschale 18 kann mittels zusätzlicher Schrauben in dem Beckenknochen 10 befestigt werden. Die Pfannenschale 18 kann auch als Schraubpfanne ausgebildet sein, die an ihrer Außenfläche ein Gewinde aufweist. Die im wesentlichen halbkugelförmige Pfannenschale 18 definiert einen Aufnahmeraum 24, der in bezug auf die Mittelachse 26 der Pfannenschale 18 rotationssymmetrisch ist.

Der Pfanneneinsatz 20 weist einen Abschnitt 22 mit sphärischer Außenfläche 28 auf, die mit einer konischen oder ebenfalls sphärischen Innenfläche 30 des Aufnahmeraums 24 korrespondiert derart, daß sich die beiden vorgenannten Flächen konzentrisch zur Rotationsachse 26 berühren. Im Bereich dieser konzentrischen Berührung ist der Krümmungsradius der Innenfläche 30 des Aufnahmeraums 24 der Pfannenschale 18 stets größer als der Krümmungsradius des sphärischen Abschnitts 22 des Pfanneneinsatzes 20, so daß dieser im Aufnahmeraum 24 der Pfannenschale 18 selbsthemmend klemmbar gehalten ist. Wenn es sich bei der Innenfläche 30 um eine sich zum Pol der Pfannenschale 18 hin konisch verjüngende Umfangsfläche handelt, ist der Krümmungsradius unendlich.

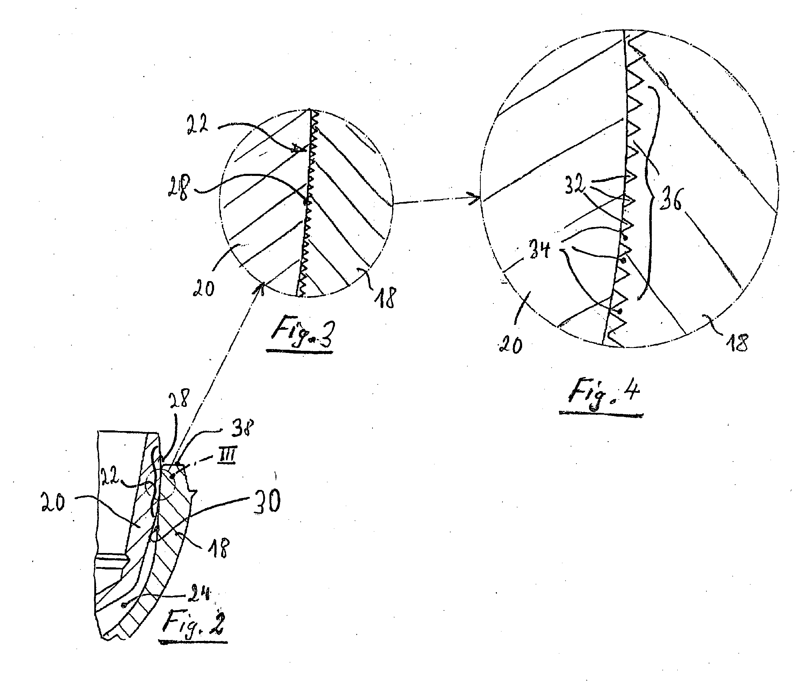

Von wesentlicher Bedeutung ist, daß der Verformungswiderstand wenigstens einer der miteinander korrespondierenden Flächen 28, 30 von Pfanneneinsatz 20 und Aufnahmeraum 24 gegenüber demjenigen des Kernbereichs dieser Teile erniedrigt ist, insbesondere durch Ausbildung einer Oberflächenstruktur. Eine solche ist bei der in den Figuren 2 bis 4 dargestellten Ausführungsform an der sphärischen Innenfläche 30 des Aufnahmeraums 24 vorgesehen, und zwar in Form von sich über den Umfang erstreckenden, und im Querschnitt dreieckigen Rillen 32, die von in Umfangsrichtung verlaufenden Rippen 34 mit ebenfalls dreieckigem Querschnitt getrennt sind. Im Bereich 36 der größten Pressung zwischen Pfanneneinsatz 20 und Pfannenschale 18 werden die Spitzen der Umfangsrippen 34 verformt, so wie dies Fig. 4 sehr deutlich erkennen läßt. Diese Spitzen sind also verantwortlich für den geringeren Verformungswiderstand der inneren Klemmfläche 30 des Aufnahmeraums 24 der Pfannenschale 18.

Die Rippen 34 erhalten durch die erwähnte Verformung einen mehr oder weniger stark ausgeprägten trapezförmigen Querschnitt.

Im Gegensatz zum Stand der Technik nach der DE 103 60 390 A1 berühren sich die Flächen 28, 30 nicht längs einer zur Rotationsachse 26 des Aufnahmeraumes 24 konzentrischen Berührungslinie, sondern längs eines zur Rotationsachse 26 des Aufnahmeraumes 24 konzentrischen Berührungsstreifens. Dadurch läßt sich der spezifische Druck zwischen den beiden vorgenannten Flächen erheblich reduzieren, ohne daß die Klemmwirkung verlorengeht. Im Gegenteil, man hat festgestellt, daß durch die beschriebenen Maßnahmen sogar eine höhere Klemmwirkung erhalten wird ohne Gefahr einer unzulässigen Verformung des Grundkörpers von Pfannenschale und/oder Pfanneneinsatz.

Es sei noch darauf hingewiesen, daß der Winkel der sich an die Äquatorebene 38 der Pfannenschale anschließende Innenfläche 30, den diese mit der Rotationsachse 26 einschließt, je nach der Materialpaarung von Pfannenschale 18 und Pfanneneinsatz 20 so gewählt ist, daß sich eine Selbsthemmung ergibt, wobei diese Selbsthemmung durch die vorgenannte Oberflächenstruktur zusätzlich erhöht wird. Vorzugsweise liegt dieser Winkel bei etwa 3,5° bis 12°, insbesondere zwischen etwa 5° und 8°. Bei einer metallischen Pfannenschale 18 ergibt sich zum Beispiel für einen metallischen Pfanneneinsatz 20 ein selbsthemmender Kegelwinkel von etwa 4,0° bis 5,0° und für einen keramischen Pfanneneinsatz 20 ein selbsthemmender Kegelwinkel von ca. 8,0° bis 10,0°.

Es sei auch noch darauf hingewiesen, daß es erfindungsgemäß ist, diejenige der beiden miteinander korrespondierenden Kontakt- bzw. Klemmflächen, die keine ausgeprägte Oberflächenstruktur aufweist, wenigstens aufzurauhen mit einem Mittelrauhwert R₂ (gemäß DIN 4760 bzw. VSM 10321), der zwischen 0,5 und 3,5 µ liegt. Dadurch erhält man eine noch bessere Verzahnung mit der strukturierten Oberfläche.

Sämtliche in den Anmeldungsunterlagen offenbarten Merkmale werden als erfindungswesentlich beansprucht, soweit sie einzeln oder in Kombination gegenüber dem Stand der Technik neu sind.

### Bezugszeichen

- 10: Beckenknochen
- 12: Prothesenschaft
- 14: Schenkelhals
- 16: Gelenkkopf
- 18: Pfannenschale
- 20: Pfanneneinsatz
- 22: sphärischer Abschnitt des Pfanneneinsatzes
- 24: Aufnahmeraum
- 26: Mittelachse (Rotationsachse) der Pfannenschale
- 28: sphärische Außenfläche des Pfanneneinsatzes
- 30: kegelförmige oder sphärische Innenfläche des Aufnahmeraums 24
- 32: Umfangsrille
- 34: Umfangsrippe
- 36: Klemmbereich des Pfanneneinsatzes innerhalb der Pfannenschale
- 38: Äquatorebene der Pfannenschale

## Patentansprüche

1. Gelenkpfanne, insbesondere für eine Hüftendoprothese, mit einer
- Pfannenschale (18), und einem
- Pfanneneinsatz (20) zur Lagerung eines Gelenkkopfes (16), wobei
- der Pfanneneinsatz (20) mit einem Abschnitt (22) mit sphärischer Außenfläche (28) in einem Aufnahmeraum (24) der Pfannenschale (18) positioniert ist, derart, daß die sphärische Außenfläche (28) des Pfanneneinsatzes (20) die Innenfläche (30) des Aufnahmeraumes (24) konzentrisch zur Rotationsachse (26) desselben berührt, und wobei
- im Bereich der konzentrischen Berührung der Krümmungsradius der Innenfläche (30) des Aufnahmeraumes (24) stets größer ist als der Krümmungsradius des sphärischen Abschnitts (22) des Pfanneneinsatzes (20), so daß dieser im Aufnahmeraum (24) der Pfannenschale (18) selbsthemmend klemmbar gehalten ist,
**dadurch gekennzeichnet, daß**
- der Verformungswiderstand einer der miteinander korrespondierenden Flächen (28, 30) von Pfanneneinsatz (20) und Aufnahmeraum (24) gegenüber demjenigen des Kernbereichs dieser Teile durch Ausbildung einer ausgeprägten Oberflächenstruktur (32, 34) erniedrigt ist und die andere der miteinander korrespondierenden Flächen (28, 30) mit einem Mittelrauhwert Rₐ gemäß DIN 4760 bzw. VSM 10321, der zwischen 0,5 und 3,5 nm liegt, aufgerauht ist.

2. Gelenkpfanne nach Anspruch 1,
**dadurch gekennzeichnet, daß**
die Oberflächenstruktur der wenigstens einen der miteinander korrespondierenden Flächen (28, 30) durch sich über den Umfang erstreckende Rippen (34) oder dgl. Erhebungen gebildet ist.

3. Gelenkpfanne nach Anspruch 2,
**dadurch gekennzeichnet, daß**
die Rippen (34) oder dgl. Erhebungen im Querschnitt dreieckförmig bzw, spitzdachartig ausgebildet sind.

4. Gelenkpfanne nach einem der Ansprüche 1 bis 3,
**dadurch gekennzeichnet, daß**
die Innenfläche (30) des Aufnahmeraums (24) im Bereich der konzentrischen Berührung konisch mit einem Krümmungsradius unendlich ausgebildet ist.

5. Gelenkpfanne nach einem der Ansprüche 1 bis 4,
**dadurch gekennzeichnet, daß**
der Winkel, den die Innenfläche (30) des Aufnahmeraums (24) mit der Rotationsachse (26) der Pfannenschale (18) einschließt, der Selbsthemmungswinkel der Materialpaarung von Pfannenschale (18) und Pfanneneinsatz (20) ist.

6. Gelenkpfanne nach Anspruch 4,
**dadurch gekennzeichnet, daß**
der Winkel der Innenfläche (30) zwischen etwa 3,5° und 12°, insbesondere zwischen etwa 5° und 8° liegt.

7. Gelenkpfanne nach einem der Ansprüche 2 bis 6,
**dadurch gekennzeichnet, daß**
die Rippen (34) oder dgl. Erhebungen als Umfangsrippen oder dgl.- erhebungen ausgebildet und jeweils über den Umfang gleichförmig verteilt unterbrochen sind.

8. Gelenkpfanne nach einem der Ansprüche 1 bis 7,
**dadurch gekennzeichnet, daß.**
die Oberflächenstruktur der wenigstens einen der miteinander korrespondierenden Flächen (28, 30) aus im Querschnitt dreieckigen Rillen (32) besteht, die von in Umfangsrichtung verlaufenden Rippen (34) mit ebenfalls dreieckigem oder gegebenenfalls auch trapezförmigem Querschnitt getrennt sind.

## Claims

1. Joint socket, especially for a hip endoprosthesis, having a
- socket shell (18), and a
- socket insert (20) for mounting a joint head (16), wherein
- the socket insert (20) is positioned by means of a portion (22) having a spherical outer face (28) in a receiving space (24) of the socket shell (18) in such a way that the spherical outer face (28) of the socket insert (20) is in contact with the inner face (30) of the receiving space (24) concentrically with the rotational axis (26) thereof, and wherein
- in the region of concentric contact, the radius of curvature of the inner face (30) of the receiving space (24) is always greater than the radius of curvature of the spherical portion (22) of the socket insert (20), so that the latter is held self-lockingly clampable in the receiving space (24) of the socket shell (18),
**characterized in that**
- the resistance to deformation of one of the mutually corresponding faces (28, 30) of socket insert (20) and receiving space (24) is reduced with respect to that of the core region of those components as a result of the formation of a prominent surface structure (32, 34), and the other of the mutually corresponding faces (28, 30) is surface-roughened with an average roughening value Rₐ according to DIN 4760 or VSM 10321 of from 0.5 to 3.5 µm.

2. Joint socket according to claim 1,
**characterized in that**
the surface structure of the at least one of the mutually corresponding faces (28, 30) is formed by ribs (34) or like protuberances extending around the periphery.

3. Joint socket according to claim 2,
**characterized in that**
the ribs (34) or like protuberances are triangular, i.e. pitched-roof-like, in cross-section.

4. Joint socket according to any one of claims 1 to 3,
**characterized in that**
the inner face (30) of the receiving space (24) in the region of concentric contact is conical with an infinite radius of curvature.

5. Joint socket according to any one of claims 1 to 4,
**characterized in that**
the angle which the inner face (30) of the receiving space (24) encloses with the rotational axis (26) of the socket shell (18) is the self-locking angle for the material pairing of socket shell (18) and socket insert (20).

6. Joint socket according to claim 4,
**characterized in that**
the angle of the inner face (30) is about from 3.5° to 12°, especially about from 5° to 8°.

7. Joint socket according to any one of claims 2 to 6,
**characterized in that**
the ribs (34) or like protuberances are in the form of peripheral ribs or like peripheral protuberances and in each case have breaks distributed uniformly around the periphery.

8. Joint socket according to any one of claims 1 to 7,
**characterized in that**
the surface structure of the at least one of the mutually corresponding faces (28, 30) consists of grooves (32) of triangular cross-section which are separated by ribs (34) running in the peripheral direction, which ribs likewise have a triangular or also possibly trapezoidal cross-section.

## Revendications

1. Cavité articulaire, plus particulièrement pour une endoprothèse de hanche, avec :
- un cotyle (18), et un
- insert de cotyle (20) pour le logement d'une tête d'articulation (16),
- l'insert de cotyle (20) étant positionné, avec une portion (22) présentant une surface externe sphérique (28), dans un espace de logement (24) du cotyle (18), de façon à ce que la surface externe sphérique (28) de l'insert de cotyle (20) entre en contact avec la surface interne (30) de l'espace de logement (24) de manière concentrique par rapport à l'axe de rotation (26) de celui-ci et
- au niveau du contact concentrique, le rayon de courbure de la surface interne (30) de l'espace de logement (24) étant toujours supérieur au rayon de courbure de la portion sphérique (22) de l'insert de cotyle (20), de façon à ce que celui-ci soit maintenu de manière autobloquante dans l'espace de logement (24) du cotyle (18),
**caractérisé en ce que**
- la résistance à la déformation d'une des surfaces en correspondance mutuelle (28, 30) de l'insert de cotyle (20) et de l'espace de logement (24) par rapport à celle de la zone centrale de ces pièces est affaiblie par la formation d'une structure de surface (32, 34) bien marquée et l'autre surface des surfaces en correspondance mutuelle (28, 30) est rendue rugueuse avec une valeur de rugosité R₂ selon DIN 4760 ou VSM 10321 entre 0,5 et 3,5 µm.

2. Cotyle selon la revendication 1, **caractérisé en ce que** la structure de la surface d'au moins une des surfaces en correspondance mutuelle (28, 30) est formée par des nervures (34) ou d'autres reliefs similaires, qui s'étendent sur la circonférence.

3. Cotyle selon la revendication 2, **caractérisé en ce que** les nervures (34) ou d'autres reliefs similaires présentent, en coupe transversale, une forme de triangle ou de toit pointu.

4. Cotyle selon l'une des revendications 1 à 3, **caractérisé en ce que** la surface interne (30) de l'espace de logement (24) présente une forme conique avec un rayon de courbure infini au niveau du contact concentrique.

5. Cotyle selon l'une des revendications 1 à 4, **caractérisé en ce que** l'angle formé par la surface interne (30) de l'espace de logement (24) avec l'axe de rotation (26) de la cupule (18) est l'angle d'autoblocage du couplage de matériaux entre la cupule (18) et l'insert de cotyle (20).

6. Cotyle selon la revendication 4, **caractérisé en ce que** l'angle de la surface interne (30) varie entre environ 3,5° et 12°, plus particulièrement entre environ 5° et 8°.

7. Cotyle selon l'une des revendications 2 à 6, **caractérisé en ce que** les nervures (34) ou autres reliefs similaires présentent la forme de nervures circonférentielles ou d'autres reliefs circonférentiels et sont interrompues de manière régulière sur la circonférence.

8. Cotyle selon l'une des revendications 1 à 7, **caractérisé en ce que** la structure de la surface d'au moins une des surfaces (28, 30) en correspondance mutuelle est constituée, en coupe transversale, de rainures triangulaires (32) séparées par des nervures (34) s'étendant dans la direction circonférentielle, et présentant également une section triangulaire ou trapézoïdale.
